# EUROPÄISCHE PATENTSCHRIFT

(11) **EP 2 436 276 B1**
(45) Veröffentlichungstag und Bekanntmachung des Hinweises auf die Patenterteilung: **28.02.2018**
(21) Anmeldenummer: 11007551.2
(22) Anmeldetag: 16.09.2011
(51) Int. Cl.: A61F 13/00, A61F 13/06, A41B 11/00, A61F 13/08, D04B 1/26

(54) **Kompressionsartikel mit Einsatz**
Compression item with insert
Article de compression doté d'un insert

(30) Priorität: 29.09.2010 DE 102010046945
(43) Veröffentlichungstag der Anmeldung: 04.04.2012
(73) Patentinhaber: BSN -Jobst GmbH, 46446 Emmerich (DE)
(72) Erfinder: Platz, Sascha, 47533 Kleeve (DE); Dittmann, Stefan, 46446 Emmerich (DE); Greve, Jürgen, 46446 Emmerich (DE)
(74) Vertreter: Kohler Schmid Möbus Patentanwälte

(56) Entgegenhaltungen:
- DE-A1- 10 358 146
- US-A- 5 211 035

## Beschreibung

Die Erfindung betrifft einen Kompressionsartikel, der aus einem elastischen Grundgestrick hergestellt ist, wobei das Grundgestrick mindestens einen gestrickten Einsatz aufweist und das Grundgestrick und der Einsatz unter Verwendung mindestens eines elastischen Strickfadens und/oder eines elastischen Schussfadens gefertigt sind.

Derartige Kompressionsartikel können beispielsweise Handschuhe, Strümpfe, Strumpfhosen oder auch Gelenkbandagen sein. Sie dienen der Stützung von Körperteilen, insbesondere Extremitäten. Der durch diese Artikel aufgebaute und therapeutisch notwendige Kompressionsdruck wird von den Trägern der Artikel in der Regel nicht als störend empfunden. Dies gilt jedoch nicht bei Kompressionsartikeln, die sich über Gelenke hinweg erstrecken. Wird das Gelenk gebeugt, so kommt es auf der Beugeinnenseite zu einer Faltenbildung, die zu Einschnürungen, Hautrötungen oder Wundscheuern der Haut führen können. Es sind daher bereits Gelenkbandagen vorgeschlagen worden, die im Gelenkbereich mit einem Einsatz versehen sind, der mit Schlitzen im Beugebereich versehen ist, um eine Faltenbildung zu vermeiden. Eine solche Bandage ist beispielsweise in der US 4,632,106 beschrieben.

Das Versehen eines Einsatzes aus einem komprimierbaren Material wie Latex zur Reduzierung der Faltenbildung ist aus der EP 0 227 566 A1 bekannt. Die DE 103 58 146 B4 beschreibt eine Bandage mit einem zweilagigen Einsatz im Beugebereich, um weichere und weniger stark einschneidende Falten zu erreichen, wobei im Beugebereich kein Schussfaden eingesetzt wird.

Bei den bekannten Kompressionsartikeln mit verminderter Faltenbildung ändert sich im Bereich des Einsatzes allerdings die Kompressionswirkung des Artikels. Bei reinen Sportbandagen kann dies hinnehmbar sein, nicht jedoch bei medizinischen Artikeln. Bei diesen muss sichergestellt sein, dass die therapeutische Wirkung des Artikels gewährleistet ist und den entsprechenden Richtlinien genügt.

Der vorliegenden Erfindung liegt daher die Aufgabe zugrunde, einen auch für medizinische Zwecke einsetzbaren Kompressionsartikel vorzuschlagen, der ein Einschnüren und Wundscheuern beim Tragen des Artikels im Beugebereich eines Gelenks vermeidet.

Die Aufgabe wird durch einen Kompressionsartikel mit den Merkmalen des Anspruchs 1 gelöst.

Durch die doppellagige Ausbildung des Kompressionsartikels bilden sich bei der Positionierung des Einsatzes im Beugebereich eines Gelenks dort weniger oder zumindest weniger scharfe Falten aus als bei einem einlagigen oder klassisch doppelflächigen Gestrick, beispielsweise einem Rechts-Rechts-Flachgestrick. Gleichzeitig bleibt der Kompressionsdruck des Grundgestricks auch im Bereich des Einsatzes erhalten. Dies kann insbesondere dadurch erreicht werden, dass die Querelastizität des Einsatzes annähernd derjenigen des Grundgestricks entspricht. Dazu werden das Grundgestrick und der mindestens eine Einsatz mit dem gleichen elastischen Schussfaden gebildet. Der elastische Schussfaden bestimmt in erster Linie die Querelastizität des Artikels und sorgt somit für den erforderlichen Kompressionsdruck. Im Bereich des Einsatzes lässt sich dabei der Schussfaden vorzugsweise zwischen den beiden Lagen des Gestricks anordnen. Die hier verwendeten Begriffe "elastischer Strickfaden", "elastischer Schussfaden" folgen dabei den Definitionen der Gütesicherungsvorschrift RAL-GZ 387/1. Die beschriebenen Quer- und Längselastizitäten werden nach den in dieser Vorschrift beschriebenen Methoden bestimmt.

Bei einer bevorzugten Ausgestaltung des Artikels sind die beiden Lagen des Einsatzes punktuell durch Maschen und/oder Fanghenkel miteinander verbunden. Durch die punktuelle Verbindung lassen sich die Schussfäden im Gestrick fixieren. Gleichzeitig bleiben aber die beiden Lagen des Gestricks gegeneinander begrenzt verschiebbar, was weiter zur Reduzierung der Faltenbildung im Beugebereich eines Gelenks führt.

Weitere Vorteile ergeben sich, wenn die innere, dem Körper des Trägers des Artikels zugewandte Fläche des mindestens einen Einsatzes unter Verwendung eines Fadenmaterials gebildet ist, das eine weichere Gestrickoberfläche erzeugt als das für die Bildung der äußeren Lage verwendete Fadenmaterial. Die sich dadurch ergebende weiche Innenfläche des Einsatzes schont die Haut des Trägers und wird außerdem als sehr angenehm empfunden. Die äußere Lage hingegen kann unter Verwendung eines scheuerfesten Fadenmaterials gebildet sein, wodurch der Artikel zumindest im Bereich des Einsatzes gegen mechanische Belastungen äußerst unempfindlich gestaltet werden kann.

Eine weitere Maßnahme zur Reduktion der Faltenbildung besteht darin, den Einsatz mit einer höheren Längselastizität auszustatten als das Grundgestrick. Dies kann beispielsweise dadurch erfolgen, dass der Einsatz mehr Maschenreihen pro Längeneinheit aufweist als das Grundgestrick.

Weitere Vorteile ergeben sich, wenn die beiden Lagen des Einsatzes jeweils unter Verwendung eines elastischen und eines unelastischen Fadens gebildet sind, wobei diese beiden Fäden jeweils plattiert verstrickt sind. Die elastischen Fäden dienen der Erzeugung der gewünschten Quer- und Längselastizität, während die unelastischen Fäden für die erforderliche Formstabilität des Artikels sorgen.

Prinzipiell können das Grundgestrick und der Einsatz in gleichen oder unterschiedlichen Bindungsarten hergestellt werden. Bei einer bevorzugten Ausgestaltung kann das Grundgestrick in einer Rechts-Rechts-Bindung und der mindestens eine Einsatz in einer doppellagigen Single-Jersey-Bindung gestrickt sein.

Weiter ist es vorteilhaft, wenn der Einsatz in das Grundgestrick eingestrickt ist, wodurch Nähte zwischen dem Einsatz und dem Grundgestrick vermieden werden können. Solche Nähte führen häufig zu Druck- und Scheuerstellen, die den Tragekomfort stark beeinträchtigen.

Zur Erzeugung therapeutisch wirksamer Kompressionsverhältnisse über den gesamten Artikel ist es weiter vorteilhaft, wenn die Schussfäden unterbrechungsfrei durch das Grundgestrick und den mindestens einen Einsatz geführt sind.

Die Fertigung der Kompressionsartikel kann auf Rund- oder Flachstrickmaschinen erfolgen. Auch bei der Fertigung auf einer Flachstrickmaschine lassen sich die Artikel als Schlauch-Rund-Gestricke fertigen, die keinerlei Nähte aufweisen und außerdem optimal an die anatomischen Gegebenheiten des Trägers angepasst werden können.

Nachfolgend wird ein bevorzugtes Ausführungsbeispiel eines Kompressionsartikels gemäß der Erfindung anhand der Zeichnung näher beschrieben.

Es zeigen:
- Fig. 1: eine schematische Darstellung eines Kompressionsstrumpfes mit Einsatz;
- Fig. 2: eine Darstellung der Bindung des Grundgestricks des Kompressionsstrumpfes aus Fig. 1;
- Fig. 3: eine Darstellung der Bindung des Einsatzes des Kompressionsstrumpfes aus Fig. 1.

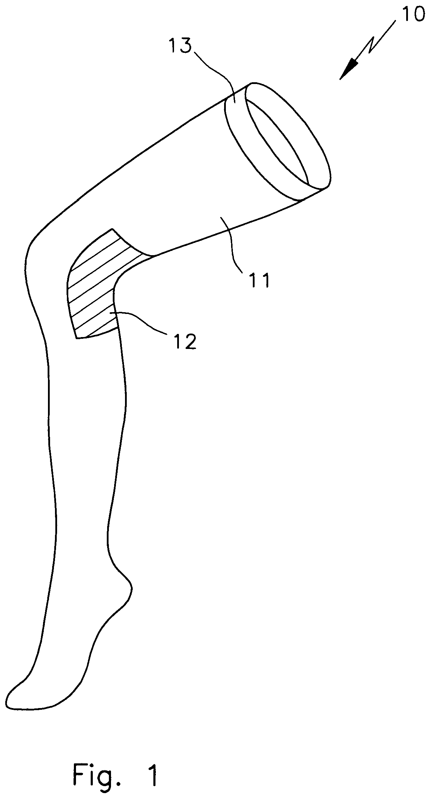

Der in Fig. 1 dargestellte Kompressionsstrumpf 10 in Form eines Kompressionsstrumpfes weist ein schlauchförmiges, anatomisch der Beinform angepasstes Grundgestrick 11 auf, das im Bereich der Kniebeuge mit einem Einsatz 12 versehen ist. Auch der Einsatz 12 ist ein Gestrick und wird vorzugsweise in das Grundgestrick 11 eingestrickt, d. h. gemeinsam mit diesem gebildet. Am oberen und unteren Rand des Kompressionsstrumpfes 10 ist ein Gestrickabschluss 13 vorgesehen.

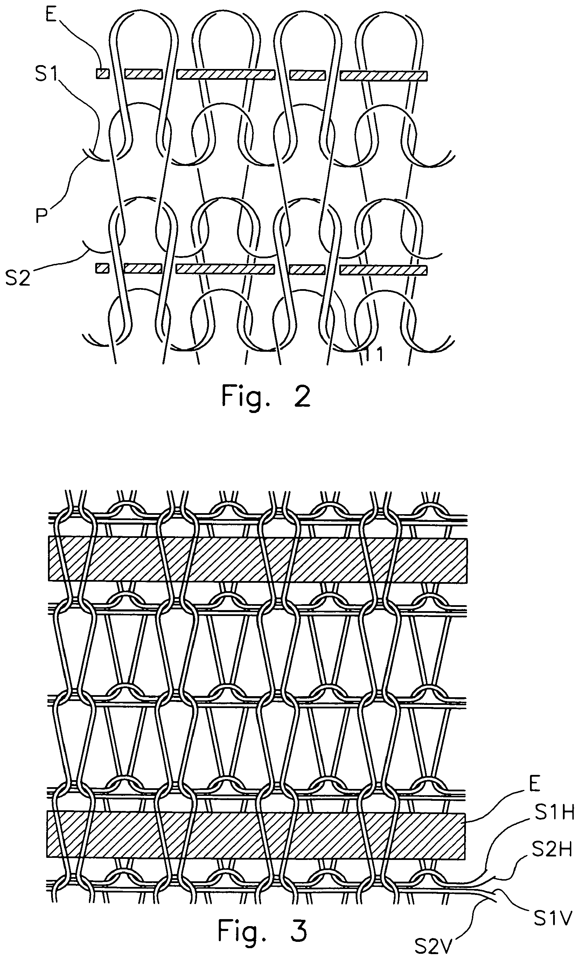

Fig. 2 zeigt eine mögliche Bindung zur Herstellung des Grundgestricks 11. Im dargestellten Beispiel ist dies eine Rechts-Rechts-Bindung, in die in jeder zweiten Maschenreihe ein Schussfaden E eingelegt ist. Der Schussfaden E ist vorzugsweise elastisch und sorgt für den erforderlichen Kompressionsdruck des Kompressionsartikels 10. Die eigentliche Rechts-Rechts-Bindung wird von zwei Strickfäden S1 und S2 gebildet, wobei im dargestellten Beispiel der Strickfaden S1 ein elastischer Faden und der Strickfaden S2 sowie der Plattierfaden P unelastische Fäden sind. Damit sorgt der Strickfaden S1 für die Längselastizität des Grundgestricks 11, während der Strickfaden S2 und der Plattierfaden P für die Formstabilität des Grundgestricks 11 sorgen.

In Fig. 3 ist eine mögliche Bindung für den Einsatz 12 gemäß der Erfindung dargestellt. Es handelt sich hierbei um ein doppellagiges Gestrick in einer Single-Jersey-Bindung, wobei der Schussfaden E, der auch für das Grundgestrick 11 verwendet wird, in jeder dritten Maschenreihe zwischen den beiden Lagen des Gestricks eingebunden wird. Zur Fixierung des Schussfadens E wird in regelmäßigen Abständen eine Masche von der hinteren Gestricklage auf die vordere Gestricklage gehängt (nicht gezeigt). Auf der Unterseite kann der Schussfaden E durch Fanghenkel gehalten werden. Durch die von hinten nach vorne umgehängten Maschen werden außerdem die beiden Lagen des Gestricks des Einsatzes 12 miteinander verbunden. Die hintere Lage bildet die innen liegende Fläche, die auf der Haut des Trägers des Kompressionsstrumpfes 10 zu liegen kommt. Sie wird durch zwei Strickfäden S1H und S2H gebildet. Der Faden S1H kann vorzugsweise ein elastischer Faden sein, während der Faden S2H nicht elastisch ist, aber eine besonders weiche, d. h. flauschige Gestrickoberfläche erzeugt. Dadurch trägt sich der Kompressionsstrumpf im Beugebereich sehr angenehm. Sich dort bildende Falten führen nicht zu Rötungen oder Wundscheuern der Haut. Die vordere und damit die außen liegende Gestricklage wird mit Strickfäden S1V und S2V gebildet. Auch hier kann der Faden S1V ein elastischer Faden und der Faden S2V ein unelastischer Faden sein. Die Fäden S1H und S1V können identisch sein. Für den Faden S2V kann ein Faden hoher Abriebsfestigkeit verwendet werden, um die Lebensdauer des Strumpfes 10 zu erhöhen.

Die Bindung in Fig. 3 ist gegenüber der Bindung des Grundgestricks 11 in Fig. 2 vergrößert dargestellt. Tatsächlich sind die Maschen des Einsatzes 12 kleiner als diejenigen des Grundgestricks. Der Schussfaden E wird durch das Grundgestrick 11 und den Einsatz 12 hindurch fortlaufend eingelegt. Da die Maschengröße der Bindung des Einsatzes 12 gemäß Fig. 3 kleiner ist als diejenige der Maschen der in Fig. 2 dargestellten Bindung des Grundgestricks 11, wird der Schussfaden E im Grundgestrick in jeder zweiten Maschenreihe eingelegt und im Einsatz 12 nur in jeder dritten. Die höhere Anzahl von Maschenreihen pro Längeneinheit im Einsatz 12 im Vergleich zum Grundgestrick 11 führt dazu, dass der Einsatz 12 eine höhere Längselastizität aufweist als das Grundgestrick 11, was das Bilden von Falten im Beugebereich des Kompressionsstrumpfes 10 weiter reduziert.

Es versteht sich, dass die in den Fig. 2 und 3 dargestellten Bindungen lediglich Beispiele sind. Es können hier auch Rechts-Links-Bindungen oder Links-Links-Bindungen eingesetzt werden. Auch die Anordnung und Auswahl der verschiedenen Strickfäden S1, S2, S1H, S2H, S1V und S2V kann unterschiedlich gestaltet werden. Außerdem kann auch die Form des Einsatzes 12 beispielsweise keilförmig sein oder aber auch sich über den gesamten Umfang des Kompressionsstrumpfes 10 erstrecken. Weiter ist die Erfindung nicht auf Kompressionsstrümpfe beschränkt, sondern erstreckt sich auf alle Arten von Kompressionsartikeln, d. h. auch Handschuhe oder Knie- oder andere Gelenkbandagen.

## Patentansprüche

1. Kompressionsartikel, der über ein Gelenk ziehbar ist und der aus einem elastischen Grundgestrick (11) hergestellt ist, wobei das Grundgestrick (11) mindestens einen gestrickten Einsatz (12) für die Beugeinnenseite des Gelenks aufweist und das Grundgestrick (11) und der Einsatz (12) unter Verwendung mindestens eines elastischen Strickfadens (S1, S2, P, S1H, S2H, S1V, S2V) und eines elastischen Schussfadens (E) gefertigt sind, **dadurch gekennzeichnet, dass** der Einsatz (12) doppellagig gestrickt ist und den gleichen Kompressionsdruck auf den Körper des Trägers des Artikels (10) ausübt wie das Grundgestrick (11), indem das Grundgestrick (11) und der mindestens eine Einsatz (12) mit dem gleichen elastischen Schussfaden (E) gestrickt sind.

2. Kompressionsartikel nach Anspruch 1, **dadurch gekennzeichnet, dass** die beiden Lagen des mindestens einen Einsatzes (12) punktuell durch Maschen und/oder Fanghenkel miteinander verbunden sind.

3. Kompressionsartikel nach einem der vorhergehenden Ansprüche, **dadurch gekennzeichnet, dass** die innere, dem Körper des Trägers des Artikels (10) zugewandte Lage des mindestens einen Einsatzes (12) unter Verwendung eines Fadenmaterials (S2H) gestrickt ist, das eine weichere Gestrickoberfläche erzeugt als das für die äußere Lage verwendete Fadenmaterial (S2V, S1V).

4. Kompressionsartikel nach einem der vorhergehenden Ansprüche, **dadurch gekennzeichnet, dass** der Einsatz (12) eine höhere Längselastizität als das Grundgestrick (11) aufweist.

5. Kompressionsartikel nach Anspruch 4, **dadurch gekennzeichnet, dass** der Einsatz (12) mehr Maschenreihen pro Längeneinheit aufweist als das Grundgestrick (11).

6. Kompressionsartikel nach einem der vorhergehenden Ansprüche, **dadurch gekennzeichnet, dass** die beiden Lagen des Einsatzes (12) jeweils unter Verwendung eines elastischen Fadens (S1V, S1H) und eines unelastischen Fadens (S2V, S2H) gebildet sind, wobei diese beiden Fäden (S1V, S2V; S1H, S2H) plattiert verstrickt sind.

7. Kompressionsartikel nach einem der vorhergehenden Ansprüche, **dadurch gekennzeichnet, dass** das Grundgestrick (11) in einer Rechts-Rechts-Bindung und der mindestens eine Einsatz (12) in einer doppellagigen Single-Jersey-Bindung gebildet sind.

8. Kompressionsartikel nach einem der vorhergehenden Ansprüche, **dadurch gekennzeichnet, dass** der Einsatz (12) in das Grundgestrick (11) eingestrickt ist.

9. Kompressionsartikel nach einem der vorhergehenden Ansprüche, **dadurch gekennzeichnet, dass** die Schussfäden (E) unterbrechungsfrei durch das Grundgestrick (11) und dem mindestens einen Einsatz (12) geführt sind.

## Claims

1. Compression item, which may be pulled over a joint and is made from an elastic basic knitted fabric (11), in which the basic knitted fabric (11) has at least a knitted insert (12) for the bend on the inside of the joint and the basic knitted fabric (11) and the insert (12) are made using at least an elastic knitting thread (S1, S2, P, S1H, S2H, S1V, S2V) and an elastic weft thread (E), **characterised in that** the insert (12) is knitted double layered and exercises the same compression pressure on the body of the wearer of the item (10) as the basic knitted fabric (11), in which the basic knitted fabric (11) and the at least one insert (12) are knitted with the same elastic weft threads (E).

2. Compression item according to claim 1, **characterised in that** both layers of the at least one insert (12) are connected to each other at points through stitches and/or tuck loops.

3. Compression item according to one of the previous claims, **characterised in that** the inner layer of the at least one insert (12) facing towards the body of the wearer of the item (10) is knitted using a thread material (S2H), which produces a softer knitted surface than the thread material (S2V, S1V) used for the outside layer.

4. Compression item according to one of the previous claims, **characterised in that** the insert (12) has a higher lengthwise elasticity than the basic knitted fabric (11).

5. Compression item according to claim 4, **characterised in that** the insert (12) has more rows of stitches per length unit than the basic knitted fabric (11).

6. Compression item according to one of the previous claims, **characterised in that** both layers of the insert (12) are made using an elastic thread (S1V, S1H) and a non-elastic thread (S2V, S2H), in which both these threads (S1V, S2V; S1H, S2H) are knitted plated.

7. Compression item according to one of the previous claims, **characterised in that** the basic knitted fabric (11) is made in a right-right bond and the at least one insert (12) in a double layered single jersey bond.

8. Compression item according to one of the previous claims, **characterised in that** the insert (12) is knitted into the basic knitted fabric (11).

9. Compression item according to one of the previous claims, **characterised in that** the weft threads (E) are led through the basic knitted fabric (11) and the at least one insert (12) without interruption.

## Revendications

1. Article de compression, qui est étirable par-dessus une articulation et qui est fabriqué à partir d'un tricot de base élastique (11), dans lequel le tricot de base (11) présente au moins un insert tricoté (12) pour le côté interne du pli de l'articulation et le tricot de base (11) et l'insert (12) sont fabriqués à l'aide d'au moins un fil à tricoter élastique (S1, S2, P, S1H, S2H, S1V, S2V) et d'un fil de trame élastique (E), **caractérisé en ce que** l'insert (12) est tricoté en deux couches et exerce la même pression de compression sur le corps du porteur de l'article (10) que le tricot de base (11), **en ce que** le tricot de base (11) et l'au moins un insert (12) sont tricotés avec le même fil de trame élastique (E).

2. Article de compression selon la revendication 1, **caractérisé en ce que** les deux couches de l'au moins un insert (12) sont reliées l'une à l'autre de manière ponctuelle par des mailles et/ou des boucles de charge.

3. Article de compression selon l'une quelconque des revendications précédentes, **caractérisé en ce que** la couche interne, tournée vers le corps du porteur de l'article (10), de l'au moins un insert (12) est tricoté à l'aide d'un matériau de fil (S2H) qui crée une surface de tricot plus douce que le matériau de fil (S2V, S1V) utilisé pour la couche externe.

4. Article de compression selon l'une quelconque des revendications précédentes, **caractérisé en ce que** l'insert (12) présente une élasticité de longueur supérieure que celle du tricot de base (11).

5. Article de compression selon la revendication 4, **caractérisé en ce que** l'insert (12) présente plus de rangées de mailles par unité de longueur que le tricot de base (11).

6. Article de compression selon l'une quelconque des revendications précédentes, **caractérisé en ce que** les deux couches de l'insert (12) sont formées respectivement à l'aide d'un fil élastique (S1V, S1H) et d'un fil non-élastique, dans lequel ces deux fils (S1V, S2V ; S1H, S2H) sont mêlés de manière plaquée.

7. Article de compression selon l'une quelconque des revendications précédentes, **caractérisé en ce que** le tricot de base (11) est formé en maille endroit-endroit et l'au moins un insert (12) est formé en maille Single Jersey en double couche.

8. Article de compression selon l'une quelconque des revendications précédentes, **caractérisé en ce que** l'insert (12) est tricoté dans le tricot de base (11).

9. Article de compression selon l'une quelconque des revendications précédentes, **caractérisé en ce que** les fils de trame (E) sont guidés sans interruption par le tricot de base (11) et l'au moins un insert (12).
